# EUROPÄISCHE PATENTSCHRIFT

(11) **EP 0 870 476 B1**
(45) Veröffentlichungstag und Bekanntmachung des Hinweises auf die Patenterteilung: **03.12.2003**
(21) Anmeldenummer: 98104691.5
(22) Anmeldetag: 16.03.1998
(51) Int. Cl.: A61B 19/00, A47C 7/00, A47C 9/02

(54) **Arztstuhl**
Doctor's chair
Chaise pour médecins

(30) Priorität: 11.04.1997 DE 19715147
(43) Veröffentlichungstag der Anmeldung: 14.10.1998
(73) Patentinhaber: Erich Edlinger KG, 72213 Altensteig (DE)
(72) Erfinder: Edlinger, Erich, 72227 Egenhausen (DE)
(74) Vertreter: Gleiss, Alf-Olav, Dr.jur. Dipl.-Ing.

(56) Entgegenhaltungen:
- DE-U- 8 914 940
- DE-U- 9 204 271
- FR-A- 1 073 618
- FR-A- 2 207 417
- US-A- 3 029 106

## Beschreibung

Die Erfindung betrifft einen Arztstuhl mit einer Sitzfläche, die von einer Sitzsäule getragen wird, die mit einem feststellbaren, auf einem Untergrund verfahrbaren Fahrgestell verbunden ist. Ein Arztstuhl gemäß dem Oberbegriff des Anspruchs 1 ist aus FR-A-1 073 618 bekannt.

Arztstühle der hier angesprochenen Art sind bekannt, auch solche, die ein feststellbares, auf einem Untergrund ruhendes beziehungsweise verfahrbares Fahrgestell aufweisen. Es hat sich herausgestellt, daß bei Präzisionsarbeiten, die beispielsweise von einem Zahn- oder Augenarzt durchgeführt werden, eine Gefährdung des Patienten dadurch gegeben ist, daß auch bei festgestelltem Fahrgestell eine Restbeweglichkeit des Arztstuhls gegeben ist. Bei einer unvorhergesehenen Bewegung des Stuhls kann der Arzt den Patienten verletzen.

Ein Arzt, insbesondere ein Zahnarzt, befindet sich während der Patientenbehandlung in einer Haltung, in der er seinen Oberkörper aus eigener Kraft in einer nach vornübergebeugten, verdrehten Position zu halten hat. Zusätzlich zu dieser angespannten Zwangshaltung, die der Arzt einnimmt, um sich in eine optimale Behandlungsposition zu bringen, verstärkt sich die Belastung des Arztes dadurch, daß dieser den verfahrbaren Arztstuhl mit seinen Füßen am Fußboden fixieren muß. Diese Gesamtbelastung führt auf Dauer unter Umständen zu erheblichen gesundheitlichen Problemen (Langzeitschäden). Diese Schäden erstrecken sich insbesondere auf die Rükken- und Halsmuskulatur, auf die Wirbelsäule, insbesondere im Bereich der Lenden- und Halswirbelsäule, den Nierenbereich, sowie auf den Schulter-, Nacken- und Kopfbereich.

Es ist daher Aufgabe der Erfindung, einen Arztstuhl der eingangs genannten Art zu schaffen, der diese Nachteile nicht aufweist.

Zur Lösung dieser Aufgabe wird ein Arztstuhl vorgeschlagen, der die in Anspruch 1 genannten Merkmale aufweist. Er zeichnet sich dadurch aus, daß mindestens eine unmittelbar mit dem Untergrund in Eingriff bringbare Feststelleinrichtung vorgesehen ist, die am Fahrgestell gelagert ist und mittels einer Betätigungseinrichtung aktivierbar und deaktivierbar ist. Dadurch, daß die Feststelleinrichtung unmittelbar auf den Untergrund einwirkt, ist eine Restbeweglichkeit des Arztstuhls im fixierten Zustand praktisch ausgeschlossen, so daß eine Gefährdung des Patienten vermieden wird.

Bevorzugt wird ein Ausführungsbeispiel des Arztstuhls, das sich dadurch auszeichnet, daß die Feststelleinrichtung ein Keilgetriebe umfaßt. Dieses ist einfach aufgebaut und damit kostengünstig realisierbar. Außerdem kann auf einfache Weise eine Feststellung des Fahrgestells gewährleistet werden, die sehr störunanfällig ist.

Bevorzugt wird eine Ausführungsform des Arztstuhls, die sich dadurch auszeichnet, daß das Fahrgestell mindestens drei vorzugsweise fünf oder sechs Rolloder Gleitelemente und vorzugsweise gleichviele Feststelleinrichtungen aufweist. Auf diese Weise ist ein sehr sicherer Halt des Arztstuhles im blokkierten Zustand gewährleistet.

Weitere Ausgestaltungen ergeben sich aus den übrigen Unteransprüchen.

Die Erfindung wird im folgenden anhand der Zeichnungen näher erläutert. Es zeigen:
- Figur 1: eine perspektivische Ansicht eines Arztstuhls;
- Figur 2: eine Seitenansicht eines Arztstuhls im Höhenschnitt;
- Figur 3: eine Prinzipskizze des Fahrgestells eines Arztstuhls und
- Figur 4: eine Detaildarstellung einer Feststelleinrichtung.

Der im folgenden beschriebene Arztstuhl ist allgemein für verschiedene Bereiche der ärztlichen Tätigkeit verwendbar. Er ist insbesondere geeignet für Zahnärzte und beispielsweise Augenärzte, also für Ärzte, die sehr feine Präzisionsarbeit leisten müssen. Im folgenden wird beispielhaft davon ausgegangen, daß es sich hier um einen Zahnarztstuhl handelt.

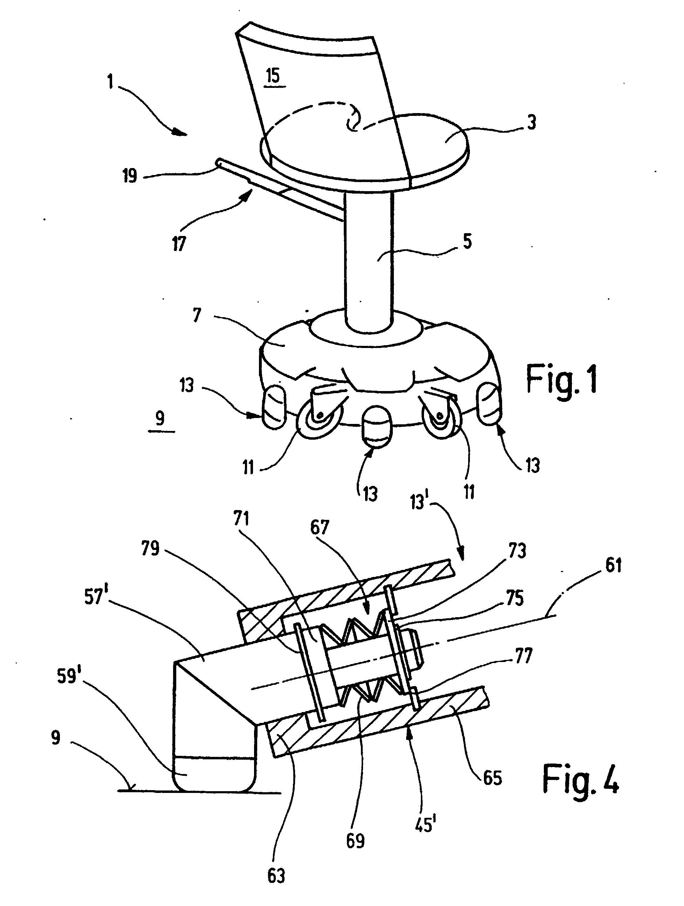

Der in Figur 1 dargestellte Arztstuhl 1 weist eine Sitzfläche 3 auf, die von einer Sitzsäule 5 getragen wird. Diese ist mit einem Fahrgestell 7 verbunden, das auf einem Untergrund 9 ruht beziehungsweise verfahrbar ist. Das Fahrgestell ist hier mit als Räder 11 ausgebildeten Rollelementen versehen. Statt dieser können auch Gleitelemente vorgesehen. werden.

An dem Fahrgestell ist mindestens eine Feststelleinrichtung 13 vorgesehen, die unmittelbar mit dem Untergrund 9 in Eingriff bringbar ist.

An der Sitzfläche ist hier beispielhaft noch eine Lehne 15 angebracht.

Deutlich erkennbar ist eine Betätigungseinrichtung 17, die zur Aktivierung und Deaktivierung'der Feststelleinrichtungen dient und beispielsweise einen Handhebel 19 umfaßt.

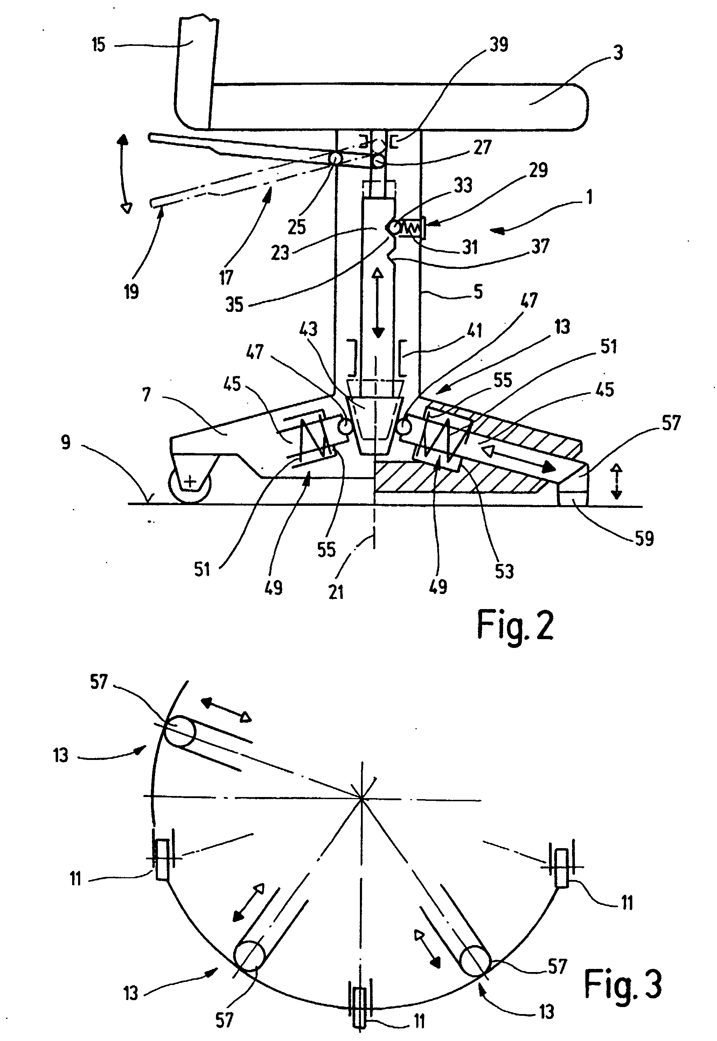

Figur 2 zeigt den in Figur 1 dargestellten Arztstuhl 1 im Höhenschnitt. Das Fahrgestell 7 ist teilweise, hier rechts einer gedachten Mittelachse 21 geschnitten wiedergegeben. Gleiche Teile sind mit gleichen Bezugsziffern versehen, so daß insofern auf die Beschreibung zu Figur 1 verwiesen wird.

In der hier gewählten Darstellung ist die Sitzsäule 5 geschnitten wiedergegeben, so daß erkennbar ist, daß die Betätigungseinrichtung 17 außer dem Handhebel 19 eine Druckachse 23 umfaßt, die mit dem Handhebel gekoppelt ist. Dieser ist um eine Schwenkachse 25 schwenkbar, die an der Sitzsäule 5 befestigt ist. Der Handhebel 19 erstreckt sich über die Schwenkachse 25 hinweg und ist mit der Druckachse 23 schwenkbar gekoppelt. Es ist auch hier ein Schwenklager 27 vorgesehen. Denkbar ist es, hier ein Langloch in der Druckachse 23 vorzusehen, um den Handhebel 19 mit der Druckachse zu koppeln, welches quer zur Längsachse der Druckachse 23 verläuft.

Bei dem hier dargestellten Ausführungsbeispiel umfaßt die Betätigungseinrichtung 17 eine Rasteinrichtung 29, die ein Federelement 31 aufweist, das auf ein als Kugel oder Stift ausgebildetes Einrastelement 33 wirkt. Dieses greift in Nuten 35 und 37 ein, die in die Außenfläche der Druckachse 23 eingebracht sind. Es sind hier zwei übereinanderliegende Nuten vorgesehen, um eine Aktivierungs- und eine Deaktivierungsposition zu definieren.

Die Druckachse 23 ist im Inneren der Sitzsäule 5 durch geeignete Lager 39 und 41 gehalten, die hier lediglich angedeutet sind und die eine in Richtung der Mittelachse 21 verlaufende Hin- und Herbewegung der Druckachse 23 ermöglichen, wie durch eine Aufund Abbewegung des dem Schwenklager 27 gegenüberliegenden freien Endes des Handhebels 19 bewirkt wird.

Die Feststelleinrichtung 13 umfaßt ein mit der Druckachse 23 gekoppeltes Keilelement 43, das einen im wesentlichen konisch ausgebildeten Mantel aufweist, der zylindrisch ausgebildet ist oder auch Einzelflächen umfaßt. Das Keilelement ist Teil eines Keilgetriebes, welches der Verlagerung von Blockierelementen 45 dient, die innerhalb des Fahrgestells hin und her beweglich gelagert sind. Die Blockierelemente verlaufen unter einem spitzen Winkel gegenüber dem Untergrund 9.

Die Blockierelemente 45 der Feststelleinrichtung 13 können an ihrem dem Keilelement zugewandten Ende mit Druckelementen 47 versehen sein, die Kugeln bezeihungsweise Gleitelemente umfassen können oder als Wälzlager ausgebildet sind. Die Druckelemente dienen einerseits der Verminderung des Verschleisses und andererseits der Reduktion der Reibungskräfte, die bei einer vertikalen Verlagerung des Keilelements 43 gegenüber den in vertikaler Richtung festgelegten Blockierelementen 45 auftritt.

Mit den Blockierelementen 45 können Rückstelleinrichtungen 49 gekoppelt sein, die die Blockierelemente 45 mit einer in Richtung auf das Keilelement 43 wirkenden Kraft beaufschlagen. Beispielhaft ist hier eine Rückholdruckfeder 51 dargestellt, die sich einerseits an einer Schulter 53 im Fahrgestell 7 und andererseits an einem starr mit dem Blockierelement 45 gekoppelten Widerlager 55 abstützt.

Die Feststelleinrichtung 13 weist an den dem Keilelement 43 gegenüberliegenden Enden der Blockierelemente 45 angeordnete Blockierstopper 57 auf, die eine im wesentlichen parallel zum Untergrund 9 verlaufende Unterseite zeigen, an der hier beispielhaft aus Gummi bestehende Stopper 59 angebracht sind.

Figur 3 zeigt eine Prinzipskizze, in der eine mögliche Anordnung der Räder 11 und der Blockierstopper 57 ersichtlich ist. Bei dem in Figur 3 wiedergegebenen Ausführungsbeispiel sind drei von insgesamt fünf Rädern und drei von insgesamt fünf Blokkierstoppern angedeutet. Es zeigt sich, daß die Räder und Blockierstopper abwechselnd vorgesehen sind, so daß zwischen je zwei Rädern je ein Blokkierstopper liegt. Vorzugsweise sind die Abstände der Räder und Blockierstopper zueinander gleich gewählt, um eine gleichmäßige Verteilung der Tragkräfte der Räder und der Feststellkräfte durch die Blockierstopper zu erreichen.

Figur 4 zeigt eine Teilvergrößerung des Endbereiches einer Feststelleinrichtung 13, nämlich des außenliegenden Endes eines Blockierelements 45'. Dieses ist hier als hohles Rohr ausgebildet, in dessen Ende ein Blockierstopper 57' eingesteckt und so gelagert ist, daß dieses gegenüber der Mittelachse 61 des Blockierelements 45' hin und her bewegbar ist. Um eine ausreichende Führung des Blokkierstoppers 57' zu gewährleisten, ist die Endwand 63 des Blockierelements 45' dicker ausgebildet als dessen Seitenwände 65. An dem im Inneren des Blokkierelements 45' gelegenen Ende des Blockierstoppers 57' ist ein Dämpfungselement 67 vorgesehen, das mindestens eine, hier eine Anzahl von Tellerfedern 69 umfaßt, die sich einerseits an einer Schulter 71 des Blockierstoppers 57' und andererseits an einem beispielsweise als Federscheibe ausgebildeten Widerlager 73 abstützt. Das Widerlager 73 wird durch einen starr mit dem im Endbereich des Blokkierstoppers 57' verbundenen Sicherungsring 75 abgestützt und ist somit nur in einer Richtung verschiebbar mit dem Blockierstopper 57' gekoppelt. Das Widerlager 73 liegt an einem als Ring ausgebildeten Sicherungselement 77 an, welches fest mit der Seitenwand 65 des Blockierelements 45 verbunden ist.

In einem Abstand zum Widerlager 73 ist auf der Außenfläche des Blockierstoppers 57' ein Anschlag 79 vorgesehen, der als Sicherungsring ausgebildet sein kann.

Im folgenden wird auf die Funktion des Arztstuhls 1 und der Feststelleinrichtung 13 näher eingegangen:

Der Betätigungshebel 19 kann aus seiner in Figur 2 mit durchgezogenen Linien dargestellten Blockierstellung in eine gestrichelt dargestellte Freigabestellung verschwenkt werden, was durch einen Doppelpfeil gekennzeichnet ist. mit einer schwarzen Pfeilspitze ist die Stellung markiert, in der sich der Handhebel 19 befindet, wenn die Feststelleinrichtung 13 blockiert beziehungsweise aktiviert ist. Mit einem weißen Pfeil ist entsprechend die Position angegeben, in der sich die Feststelleinrichtung 13 in ihrer deaktivierten Stellung befindet.

Bei einer Verschwenkung des Handhebels 19 in seine Deaktivierungsposition wird die Druckachse 23 entlang der Mittelachse 21 senkrecht noch oben angehoben und befindet sich damit in ihrer gestrichelt dargestellten Entriegelungs- beziehungsweise Deaktivierungsposition. Gleichzeitig wird das starr mit der Druckachse 23 verbundene Keilelement 43 in seine gestrichelt dargestellte angehobene Position verlagert. Dadurch gelangt ein Abschnitt des Keilelements in Eingriff mit den Druckelementen 47 der Blockierelemente 45, der einen kleineren Außendurchmesser aufweist. Die Rückholdruckfedern 51 können damit die Blockierelemente 45 in Richtung der Mittelachse 21 verlagern. Da die Blockierelemente 45 unter einem spitzen Winkel zum Untergrund 9 verlaufen, werden bei einer derartigen Verlagerung, die auch hier durch einen Doppelpfeil innerhalb der Blockierelemente 45 angedeutet ist, die Blockierstopper 57 angehoben, so daß sie den Untergrund 9 nicht mehr berühren. Die Bewegung der Blokkierstopper ist ebenfalls durch einen Doppelpfeil gekennzeichnet. Wie auch bei den anderen Pfeilen ist hier durch eine ausgemalte, schwarze Pfeilspitze die Blockierstellung und durch eine weiße Pfeilspitze die Freigabestellung gekennzeichnet.

Die Freigabestellung ist dadurch gekennzeichnet, daß die Blockierstopper 57 beziehungsweise deren Stopper 59 den Untergrund 9 nicht mehr oder nur so wenig berühren, daß eine Bewegung beziehungsweise Verfahrbarkeit des Arztstuhls 1 gegenüber dem Untergrund 9 möglich ist.

Wird also der Handhebel 19 der Betätigungseinrichtung 17 von der Bedienungsperson in die gestrichelte, untere Position verlagert, so werden die Stopper 59 durch die Rückholfedern 51 angehoben. Der Arzt kann nun den Stuhl in die gewünschte Position gegenüber dem Patienten verlagern und dabei sowohl eine translatorische als auch eine rotatorische Bewegung mit dem Arztstuhl 1 durchführen.

Wird nach Erreichen der gewünschten Position der Handhebel 19 in die obere mit durchgezogenen Linien dargestellte Position verlagert, so wird auch die Druckachse 23 senkrecht nach unten gedrückt und gemeinsam mit dieser das Keilelement 43. Dieses drängt nach Art eines Keilgetriebes die Blockierelemente 45 synchron nach außen, so daß die Blokkierstopper 57 mit den Stopperen 59 nach unten in Richtung zum Untergrund 9 bewegt und auf diesen angedrückt werden. Bei Aktivierung der Stopper 59 der Feststelleinrichtung 13 wird der Arztstuhl 1 unverrückbar am Untergrund 9 fixiert, so daß der Arzt ohne Gefährdung für den Patienten die Behandlung durchführen kann.

Bei dem in Figur 4 dargestellten Ausführungsbeispiel wird der Stopper 59' der Feststelleinrichtung 13' mit einer durch das Dämpfungselement 67 begrenzten Kraft auf den Untergrund 9 angedrückt. Wird eine durch die Tellerfedern 69 vorgegebene bestimmte Maximalkraft überschritten, so kann sich der Blockierstopper 57' gegenüber dem Blockierelement 45 verschieben. Auf diese Weise kann der Verschleiß der Stopper 59' bei einem sicheren Halt des Arztstuhls 1 begrenzt werden. Überdies ist es möglich, Unebenheiten des Untergrundes 9 mit Hilfe derartiger Dämpfungselemente 67 auszugleichen. Es wird im übrigen hier eine sogenannte Endlagendämpfung erreicht, die den Bedienungskomfort erhöht.

Nach allem wird deutlich, daß Dämpfungselemente der hier angesprochenen Art auch an den den Blockierstoppern 57' gegenüberliegenden Enden der Blockierelemente 45' vorgesehen werden können. Schließlich ist es auch noch möglich, Dämpfungselemente der hier beschriebenen Art mit der Druckachse 23 zu kombinieren, so daß diese das Keilelement 43 mit einer gewissen Nachgiebigkeit gegen die Druckelemente 47 der Blockierelemente 45 anpreßt.

Nach allem wird deutlich, daß die hier gegebene Funktion der Feststelleinrichtung 13 auch bei einer gewissen Abwandlung der hier gegebenen Teilelemente sichergestellt ist. Es ist beispielsweise auch möglich, die Betätigungseinrichtung mit einem Fußhebel zu kombinieren, der das Keilelement 43 mehr oder weniger anhebt um die Blockierelemente 45 mehr oder weniger weit gegenüber der Mittelachse 23 nach außen zu verlagern und die Feststelleinrichtung 13 zu aktivieren. Denkbar ist es auch noch, den Handhebel 19 an seinem dem Betätigungsende gegenüberliegenden Ende an der Sitzsäule 5 schwenkbar anzubringen. Wird nun der Handhebel nach oben bewegt, so wird auch die Druckachse 23 senkrecht noch oben angehoben. Die Aktivierungs- und Deaktivierungsstellung des Handhebels 19 wird auf diese Weise umgekehrt gegenüber den in Figur 2 dargestellten Positionen.

## Patentansprüche

1. Arztstuhl mit einer Sitzfläche, die von einer Sitzsäule getragen wird, die mit einem feststellbaren, auf einem Untergrund verfahrbaren Fahrgestell verbunden ist, und mit mindestens einer unmittelbar mit dem Untergrund (9) in Eingriff bringbaren Feststelleinrichtung, die am Fahrgestell (7) gelagert ist und mittels einer Betätigungseinrichtung (17) aktivier- und deaktivierbar ist, **dadurch gekennzeichnet, dass** die Feststelleinrichtung (13) ein Keilgetriebe und mindestens ein schräg zur Mittelachse (21) der Sitzsäule verlagerbares Blockierelement (45,45') umfasst, das einen Blockierstopper (59,59') aufweist.

2. Arztstuhl nach Anspruch 1, **dadurch gekennzeichnet, daß** das Keilgetriebe ein im wesentlichen konzentrisch zur Sitzsäule (5) angeordnetes Keilelement (43) umfaßt.

3. Arztstuhl nach einem der vorhergehenden Ansprüche, **dadurch gekennzeichnet, daß** die Betätigungseinrichtung (17) einen über eine Druckachse (23) mit dem Keilelement (43) gekoppelten Handhebel (19) umfaßt.

4. Arztstuhl nach einem der vorhergehenden Ansprüche, **dadurch gekennzeichnet, daß** innerhalb des Fahrgestells (7) ein Blockierelement (45,45') verlagerbar ist.

5. Arztstuhl nach Anspruch 4, **dadurch gekennzeichnet, daß** das Blockierelement (45,45') unter einem vorzugsweise spitzen Winkel zum Untergrund (9) verläuft.

6. Arztstuhl nach einem der vorhergehenden Ansprüche, **dadurch gekennzeichnet, daß** das Blockierelement (45,45') ein an einem Ende des Blockierelements angeordneten Blockierstopper (59,59') umfaßt.

7. Arztstuhl nach Anspruch 6, **dadurch gekennzeichnet, daß** der Blockierstopper (59') federnd gelagert ist.

8. Arztstuhl nach einem der vorhergehenden Ansprüche, **dadurch gekennzeichnet, daß** das Blockierelement (45,45') an seinem dem Blockierstopper (59,59') gegenüberliegenden Ende ein mit dem Keilelement (43) zusammenwirkendes Druckelement (47) aufweist.

9. Arztstuhl nach einem der vorhergehenden Ansprüche, **dadurch gekennzeichnet, daß** das Fahrgestell (7) mindestens drei, vorzugsweise fünf oder sechs Roll- oder Gleitelemente (Räder (11)) aufweist.

10. Arztstuhl nach Anspruch 9, **dadurch gekennzeichnet, daß** zwischen je zwei Roll- oder Gleitelementen eine Feststelleinrichtung angeordnet ist.

## Claims

1. A doctor's chair with a seat surface which is carried by a seat column which is connected to a carriage which can be located and which can travel on a floor, and with at last one locating means which can be brought into direct contact with the floor (9), which is mounted on the carriage (7) and which can be engaged and disengaged by means of an actuating device (17), **characterized in that** the locating means (13) comprises a wedge mechanism and at least one locking member (45, 45') which can be displaced diagonally to the centre axis (21) of the seat column and which has a locking stop (59, 59').

2. A doctor's chair according to claim 1, **characterized in that** the wedge mechanism comprises a wedge member (43) disposed substantially concentrically to the seat column (5).

3. A doctor's chair according to any one of the preceding claims, **characterized in that** the actuating device (17) comprises a hand lever (19) coupled to the wedge member (43) via a compression shaft (23).

4. A doctor's chair according to any one of the preceding claims, **characterized in that** inside the carriage (7) there is a displaceable locking member (45, 45').

5. A doctor's chair according to claim 4, **characterized in that** the locking member (45, 45') extends at a preferably acute angle relative to the floor (9).

6. A doctor's chair according to any one of the preceding claims, **characterized in that** the locking member (45, 45') comprises a locking stop (59,59') disposed at one end of the locking member.

7. A doctor's chair according to claim 6, **characterized in that** the locking stop (59') is spring-mounted.

8. A doctor's chair according to any one of the preceding claims, **characterized in that** at its end opposite the locking stop (59, 59') the locking member (45, 45') has a compression element (47) co-operating with the wedge member (43).

9. A doctor's chair according to any one of the preceding claims, **characterized in that** the carriage (7) has at least three, preferably five or six, rolling or sliding elements (wheels (11)).

10. A doctor's chair according to claim 9, **characterized in that** a locating means is provided between each two rolling or sliding elements.

## Revendications

1. Chaise de médecin comprenant une assise supportée par une colonne reliée à un châssis pouvant être déplacé sur le sol et pouvant être immobilisé, et au moins un dispositif d'immobilisation pouvant être indirectement mis en prise avec le sol (9), qui est monté sur le châssis (7) qui peut être activé et désactivé au moyen d'un dispositif d'actionnement (17), **caractérisée en ce que** le dispositif d'immobilisation (13) comprend un mécanisme de calage et au moins un élément de blocage (45, 45') pouvant être déplacé en biais par rapport à l'axe médian (21) de la colonne du siège, qui présente un frein (59, 59').

2. Chaise de médecin selon la revendication 1, **caractérisée en ce que** le mécanisme de calage comprend un élément de calage (43) disposé essentiellement de façon concentrique par rapport à la colonne du siège (5).

3. Chaise de médecin selon l'une des revendications précédentes, **caractérisée en ce que** le dispositif d'actionnement (17) comprend un levier à main (19) accouplé à l'élément de calage (43) par l'intermédiaire d'un arbre de poussée.

4. Chaise de médecin selon l'une des revendications précédentes, **caractérisée en ce que** à l'intérieure du châssis (7) est un élément de blocage (45, 45') déplaçable.

5. Chaise de médecin selon la revendication 4, **caractérisée en ce que** l'élément de blocage (45, 45') s'étend de préférence avec un angle aigu par rapport au sol (9).

6. Chaise de médecin selon l'une des revendications précédentes, **caractérisée en ce que** l'élément de blocage (45, 45') comporte un frein (59, 59') situé à une extrémité dudit élément de blocage.

7. Chaise de médecin selon la revendication 6, **caractérisée en ce que** le frein (59') est monté sur ressort.

8. Chaise de médecin selon l'une des revendications précédentes, **caractérisée en ce que** l'élément de blocage (45, 45') présente à son extrémité opposée au frein (59, 59') un élément de pression (47) coopérant avec l'élément de calage (43).

9. Chaise de médecin selon l'une des revendications précédentes, **caractérisée en ce que** le châssis (7) comporte au moins trois, de préférence cinq ou six éléments de roulement ou de glissement (roues (11)).

10. Chaise de médecin selon la revendication 9, **caractérisée en ce qu'**un dispositif d'immobilisation est chaque fois placé entre deux éléments de roulement ou de glissement.
